# EUROPÄISCHE PATENTSCHRIFT

(11) **EP 1 919 493 B1**
(45) Veröffentlichungstag und Bekanntmachung des Hinweises auf die Patenterteilung: **30.07.2014**
(21) Anmeldenummer: 06776693.1
(22) Anmeldetag: 09.08.2006
(51) Int. Cl.: A61K 38/05, A61P 1/18

(54) **VERWENDUNG VON SPEZIFISCHEN TRIFLUORMETHYLKETONEN ZUR VORBEUGUNG UND BEHANDLUNG EINER PANKREATITIS**
USE OF SPECIFIC TRIFLUOROMETHYL KETONES FOR PREVENTING AND TREATING PANCREATITIS
UTILISATION DE TRIFLUOROMETHYLCETONES SPECIFIQUES POUR PREVENIR ET TRAITER UNE PANCREATITE

(30) Priorität: 10.08.2005 DE 102005037791
(43) Veröffentlichungstag der Anmeldung: 14.05.2008
(73) Patentinhaber: Lerch, Markus M., 17489 Greifswald (DE); Mayerle, Julia, 17489 Greifswald (DE)
(72) Erfinder: Lerch, Markus M., 17489 Greifswald (DE); Mayerle, Julia, 17489 Greifswald (DE)
(74) Vertreter: UEXKÜLL & STOLBERG
(86) Internationale Anmeldenummer: PCT/EP2006/007859
(87) Internationale Veröffentlichungsnummer: WO 2007/017259

(56) Entgegenhaltungen:
- EP-A- 1 473 302
- VEALE CHRIS A ET AL: "Orally active trifluoromethyl ketone inhibitors of human leukocyte elastase" JOURNAL OF MEDICINAL CHEMISTRY, Bd. 40, Nr. 20, 1997, Seiten 3173-3181, XP009088384 ISSN: 0022-2623
- MAYERLE J ET AL: "[Chronic pancreatitis. Diagnosis and treatment]" DER CHIRURG; ZEITSCHRIFT FÜR ALLE GEBIETE DER OPERATIVEN MEDIZEN JUL 2004, Bd. 75, Nr. 7, Juli 2004 (2004-07), Seiten 731-747 ; qui, XP002449997 ISSN: 0009-4722
- MAYERLE JULIA ET AL: "[Conservative treatment of acute pancreatitis]" MEDIZINISCHE KLINIK (MUNICH, GERMANY : 1983) 15 DEC 2003, Bd. 98, Nr. 12, 15. Dezember 2003 (2003-12-15), Seiten 744-749, XP002449998 ISSN: 0723-5003

## Beschreibung

Die vorliegende Erfindung betrifft die Verwendung von spezifischen Trifluormethylketonen zur Vorbeugung und Behandlung einer Pankreatitis und insbesondere der chronischen Pankreatitis.

Sowohl die chronische als auch die akute Pankreatitis sind häufige Erkrankungen des Gastrointestinaltraktes mit einer hohen sozioökonomischen Bedeutung.

Die chronische Pankreatitis ist mit einer Inzidenz von 8,2 Fällen/100.000 Einwohner und einer Prävalenz von 27,4 Fällen eine der häufigen Erkrankungen des Gastrointestinaltraktes. Die häufigste Ursache für eine chronische Pankreatitis ist der Alkoholmißbrauch.

Die hereditäre Pankreatitis ist eine autosomal-dominant vererbbare Form der chronischen Pankreatitis, mit einer phänotypischen Penetranz von bis zu 80%. Sie wird vor allem durch eine Mutation im PRSS1 Gen, das für kationisches Trypsinogen kodiert, ausgelöst (Withcomb et al., 1996). Sie ist durch rezidivierende Pankreatitisschübe, die meist im frühen Kindesalter beginnen, durch eine meist positive Familienanamnese, eine weitgehend gleiche Geschlechtsverteilung sowie das Fehlen anderer krankheitsassoziierter Risikofaktoren charakterisiert.

Die chronische Pankreatitis ist eine schubweise verlaufende, nichtinfektiöse Entzündung der Bauchspeicheldrüse. Sie kann mit fokalen Nekrosen, entzündlichen Infiltraten, Fibrose des Parenchyms, Steinbildung in den Gängen und der Bildung von Pseudozysten einhergehen. In fortgeschrittenen Stadien kommt es zu einer globalen Funktionseinschränkung mit herabgesetzter exokriner und endokriner Pankreasfunktion bis hin zur exokrinen und endokrinen Pankreasinsuffizienz (pankreopriver Diabetes mellitus) (Ammann et al., 1984).

Das Leitsymptom der chronischen Pankreatitis ist ein gürtelförmiger Oberbauchschmerz, ein Gewichtsverlust, verbunden mit einer Steatorrhoe, und der Diabetes mellitus. Die Diagnose wird im Allgemeinen durch bildgebende Verfahren wie den transabdominalen Ultraschall und die ERCP oder durch die Untersuchung der Pankreasfunktion gestellt.

Die Therapie der chronischen Pankreatitis beschränkt sich bei fehlenden kausalen Therapieansätzen auf die Symptombekämpfung. Behandlungsziele der chronischen Pankreatitis sind die Kompensation der exokrinen Pankreasinsuffizienz und damit die Symptombehandlung der Maldigestion, Steatorrhoe und des Gewichtsverlusts, die Behandlung der diabetischen Stoffwechsellage und eine adäquate Schmerztherapie.

Für die Schmerztherapie werden peripher wirksame Analgetika empfohlen, die in der 2. Stufe mit Neuroleptika oder Tramadolsulphat kombiniert werden können. Die 3. Stufe sieht die Verordnung von potenten zentral wirksamen Opioiden vor. Für die Behandlung des pankreopriven Diabetes mellitus sollte frühzeitig eine Insulintherapie erwogen werden. Beim Auftreten eines Gewichtsverlustes von mehr als 10% des Körpergewichts, einer Steatorrhoe mit Stuhlfettausscheidungen von mehr als 15 g/Tag, dyspeptischen Beschwerden mit starkem Meteorismus oder Diarrhoe ist die Indikation zur Substitution mit Pankreasenzymen gegeben. Die meisten Enzympräparate enthalten einen pulverisierten Extrakt aus dem Schweinepankreas mit den Hauptkomponenten Lipase, Amylase, Trypsin und Chymotrypsin. Die Präparate werden in Form von mikrosphärisch verkapselten Formulierungen verabreicht. Etwa 30-60% der Patienten entwickeln Komplikationen ihrer Erkrankung wie Strikturen des Ductus hepatocholedochus, entzündliche Raumforderungen, Pankreaspseudozysten oder Pankreasgangsteine, die eine interventionelle oder operative Therapie erfordern.

Bei der ERCP (ERCP: Endoskopisch Retrograde Cholangiopankreatographie) handelt es sich um eine Darstellung der Gallenwege und des Pankreasgangsystems. Nach einer solchen ERCP entwickelt sich in 3-6% der Fälle eine Pankreatitis. Diese Untersuchungs-assozierte Komplikation zeigt eine Mortalität von etwa 1 Promille. Es konnte gezeigt werden, daß eine prophylaktische Behandlung mit einem Protease-Inhibitor, der Trypsin, Kalikrein und Plasmin hemmt und per Infusion verabreicht wurde, die Schädigung des Pankreas, hervorgerufen durch ERCP, reduzieren konnte (Cavallini et al., 1996).

Bei der chronisch rezidivierenden Pankreatitis handelt es sich um eine mit schubweisen, schmerzhaften und entzündlichen Episoden wiederkehrende Verlaufsform entweder der akuten oder chronischen Pankreatitis. Ursächlich zu Grunde liegen können: die auslösenden Faktoren' der akuten Pankreatitis oder chronischen Pankreatitis. Der Langzeitverlauf und die üblicherweise auftretenden Komplikationen entsprechen meist der chronischen Pankreatitis.

Vor diesem Hintergrund liegt die Aufgabe der vorliegenden Erfindung darin, Verbindungen bereitzustellen, die sich für die Vorbeugung (Prophylaxe) und Behandlung (Therapie) einer Pankreatitis, insbesondere der chronischen Pankreatitis, der post-ERCP-Pankreatitis und der chronisch rezidivierenden Pankreatitis, eignen. Hierfür ist eine einfache Form der Verabreichung sowie eine Reduzierung der Nebenwirkungen entscheidend.

Erfindungsgemäß wird die Aufgabe durch den Gegenstand der Ansprüche 1 bis 8 gelöst. Es wurde überraschenderweise gefunden, dass Peptidyl-Trifluormethylketone und ihre Solvate zur Behandlung der Pankreatitis, insbesondere der chronischen Pankreatitis, der chronisch rezidivierenden Pankreatitis und der post-ERCP-Pankreatitis, besonders geeignet sind. Als Solvat kann beispielsweise eine hydratisierte Form vorkommen. Diese kann beispielsweise als geminales Diol der Trifluorketongruppe existieren. Solvate können aber ebenfalls in einer Form vorkommen, die Wassermoleküle als Teil des Kristallgitters enthält.

Die Erfindung betrifft somit die Verwendung von Peptidyl-Trifluormethylketonen oder ihrer Solvate zur Herstellung einer pharmazeutischen Zusammensetzung zur Vorbeugung und Behandlung einer Pankreatitis.

Das Peptidyl-Trifluormethylketon ist die Verbindung I, d.h. 1-[2-(4-Methoxybenzamid)-3-methylbutyryl]-N-[2-methyl-1-(trifluoracetyl)propyl]pyrrolidin-2-carboxamid der Formel 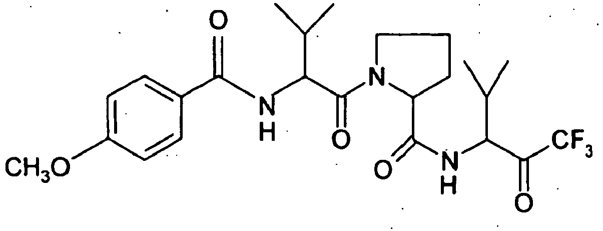 die man zur Herstellung einer pharmazeutischen Zusammensetzung zur Vorbeugung und Behandlung einer Pankreatitis verwendet.

In einer besonders bevorzugten Ausführungsform der Erfindung ist das Peptidyl-Trifluormethylketon die Verbindung II 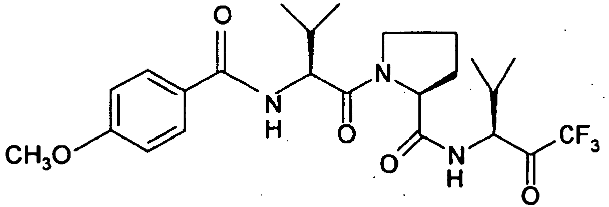 (In einer Ausführungsform ist die Pankreatitis eine nicht-akute)
Pankreatitis, eine ERCP-induzierte Pankreatitis oder eine chronische oder chronische rezidivierende Pankreatitis.

Herstellungsverfahren für die erfindungsgemäßen Verbindungen sind in der internationalen Anmeldung WO 95/21855 beschrieben, auf die hiermit ausdrücklich Bezug genommen wird. So werden beispielsweise auf den Seiten 8-16 der WO 95/21855 verschiedene Verfahren zur Herstellung der Verbindung II beschrieben.

Die Verbindung II ist das sogenannte S,S,S-Stereoisomer der Verbindung I und liegt gemäß einer Ausführungsform der Erfindung in einer Form vor, die einen Anteil von höchstens 10% der anderen möglichen Stereoisomere der Verbindung I enthält. In einer bevorzugten Ausführungsform beträgt dieser Anteil höchstens 5% und in einer besonders bevorzugten Ausführungsform höchstens 2%.

Aus WO 95/21855 ist bekannt, daß die Verbindung II zur Behandlung von Atemwegserkrankungen eingesetzt werden kann.

Überraschenderweise wurde im Rahmen der vorliegenden Erfindung festgestellt, dass sich die erfindungsgemäßen Verbindungen auch für eine prophylaktische Behandlung der Pankreatitis, insbesondere der chronischen Pankreatitis, der chronisch rezidivierenden Pankreatitis und der post-ERCP-Pankreatitis, eignen. Damit stehen zur Behandlung der vorgenannten Erkrankungen erstmals Wirkstoffe zur Verfügung, die sich oral verabreichen lassen und daher in besonderer Weise für eine Prophylaxe geeignet sind.

In einer bevorzugten Ausführungsform wird die Verbindung I und in einer besonders bevorzugten Ausführungsform die Verbindung II also zur Herstellung einer pharmazeutischen Zusammensetzung zur prophylaktischen Behandlung der chronischen Pankreatitis eingesetzt. Die pharmazeutischen Zusammensetzungen können dabei durch intravenöse Injektion oder' intraperitoneal verabreicht werden. In einer bevorzugten Ausführungsform sind die pharmazeutischen Zusammensetzungen zur oralen Verabreichung formuliert. Die orale Bioverfügbarkeit macht die pharmazeutischen Zusammensetzungen besonders geeignet für eine dauerhafte prophylaktische Behandlung.

Für eine prophylaktische Behandlung einer chronischen Pankreatitis oder der chronisch rezidivierenden Pankreatitis enthalten die pharmazeutischen Zusammensetzungen das entsprechende Peptidyl-Trifluormethylketon in einer Menge von etwa 10 bis 240 mg/kg Körpergewicht, wenn die Zusammensetzung täglich verabreicht wird.

Die pharmazeutische Zusammensetzung kann für eine prophylaktische Behandlung einer chronischen Pankreatitis oder der chronisch rezidivierenden Pankreatitis dauerhaft verabreicht werden, wobei die Zusammensetzung in einer bevorzugten Ausführungsform dreimal täglich, beispielsweise Morgens, Mittags und Abends, oral verabreicht wird. Die biologische Halbwertszeit der Verbindung beträgt in Ratte, Hamster und Hund zwischen 0,82 und 2 Stunden. Die intravenöse AUC (Bioverfügbarkeit gemessen als das Verhältnis von intravenöser zu oraler Gabe als Fläche unter der Kurve (AUC)) [ng*h/ml] liegt zwischen 1365 und 3179 ng*h/ml(Bioverfügbarkeit gemessen als das Verhältnis von intravenöser zu oraler Gabe als Fläche unter der Kurve (AUC)) Die orale Bioverfügbarkeit liegt zwischen 355 bis 1400 ng*h/ml. Die prozentuale Bioverfügbarkeit wurde bestimmt mit 26-82%. Die orale Applikation von 10mg/kg Körpergewicht der Verbindung II führt zu einer 84% Inhibition der neutrophilen Elastase. Zur Hemmung von 50% der Enzymaktivität müssen bei oraler Gabe 4,9 mg/kg Körpergewicht eingesetzt werden, hingegen nur 0,59 mg/kg Körpergewicht bei intravenöser Gabe.

In einer weiteren Ausführungsform wird die Verbindung I oder die Verbindung II zur Herstellung einer pharmazeutischen Zusammensetzung für die prophylaktische Behandlung vor einer ERCP-Untersuchung verwendet, d.h. zur Verhinderung einer post-ERCP Pankreatitis.

Für die Verhinderung einer ERCP-induzierten Pankreatitis wurden verschiedenste Ansätze vorgeschlagen, wie zum Beispiel die prophylaktische Gabe von Aprotinin, Glukagon, Calcitonin, Nifedipin oder Somatostatin, die jedoch alle erfolglos waren. Überraschenderweise eignen sich die erfindungsgemäßen Verbindungen für eine wirksame prohylaktische Behandlung einer ERCP-induzierten Pankreatitis.

Die pharmazeutischen Zusammensetzungen können vor einer ERCP per Infusion verabreicht werden. In einer bevorzugten Ausführungsform wird die pharmazeutische Zusammensetzung vor einer ERCP oral verabreicht. Dabei kann die Menge der entsprechenden Verbindung (bevorzugt Verbindung I oder II) in einem Bereich von 10 bis 240 mg/kg Körpergewicht variieren. In einer bevorzugten Ausführungsform beträgt die verwendete Menge 30 bis 120 mg/kg Körpergewicht. Es erfolgt eine einmal Gabe 30 Minuten vor der ERCP, entweder als orale Gabe oder als intravenöse Gabe in einer 10-fach niedrigeren Konzentration.

In einer weiteren bevorzugten Ausführungsform der Erfindung umfassen die erfindungsgemäßen Verwendungen ferner den Einsatz eines pharmazeutisch geeigneten Trägermaterials.

Die Erfindung wird nachfolgend anhand von Beispielen und Abbildungen beschrieben. Die Beispiele sind zur Veranschaulichung der Erfindung gedacht, sollen die Erfindung aber keineswegs beschränken.

### Beschreibung der Abbildungen:

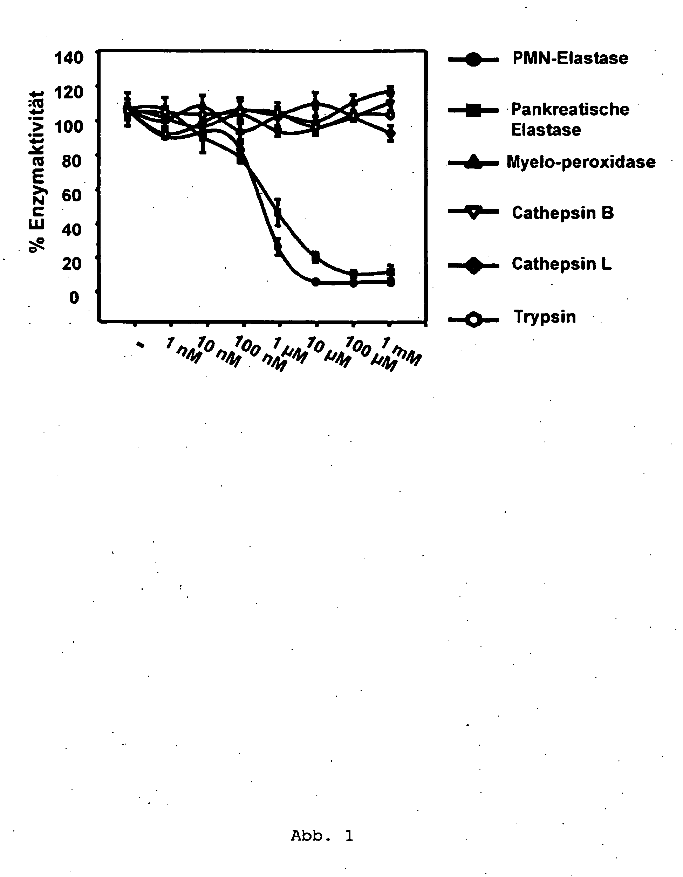

**Abbildung 1** zeigt die inhibitorische Wirkung der Verbindung II. Die humane neutrophile Elastase und in einem etwas niedrigeren Maße auch die pankreatische Elastase wird durch die Verbindung II mit einer 50%-Inhibition bei 50 nM (neutrophile Elastase) und 1 µM (pankreatische Elastase) inhibiert. Trypsin, Myeloperoxidase, Cathepsin B und L werden durch die Inkubation mit der Verbindung II nicht signifikant beeinflußt. Kᵢ neutrophile Elastase 6.7 nM, Kᵢ pankreatische Elastase 200 nM.

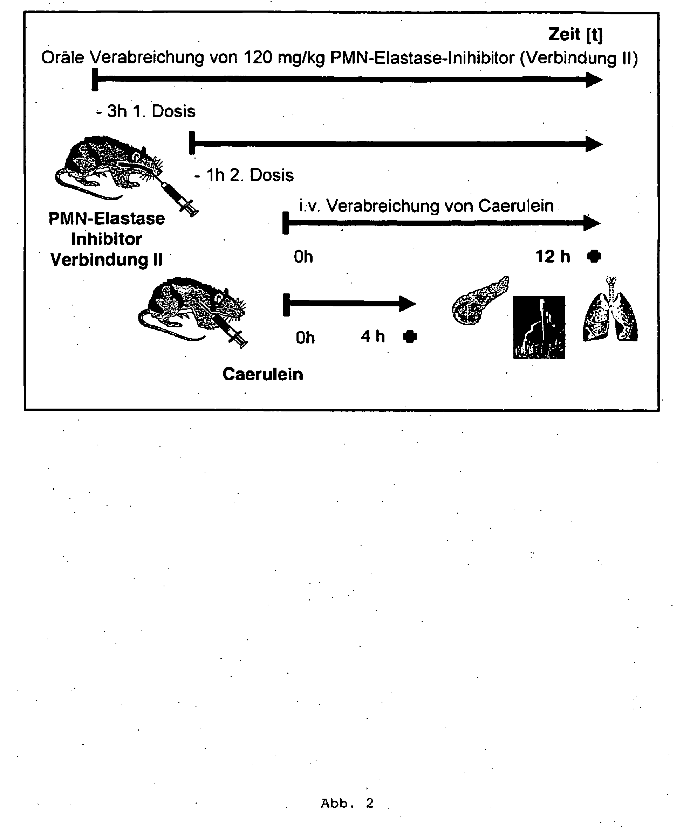

**Abbildung 2** zeigt den Aufbau eines Experimentes um die Fähigkeit der Verbindung II zu untersuchen, und die lokalen und systemischen Schäden während einer akuten Pankreatitis zu verringern.

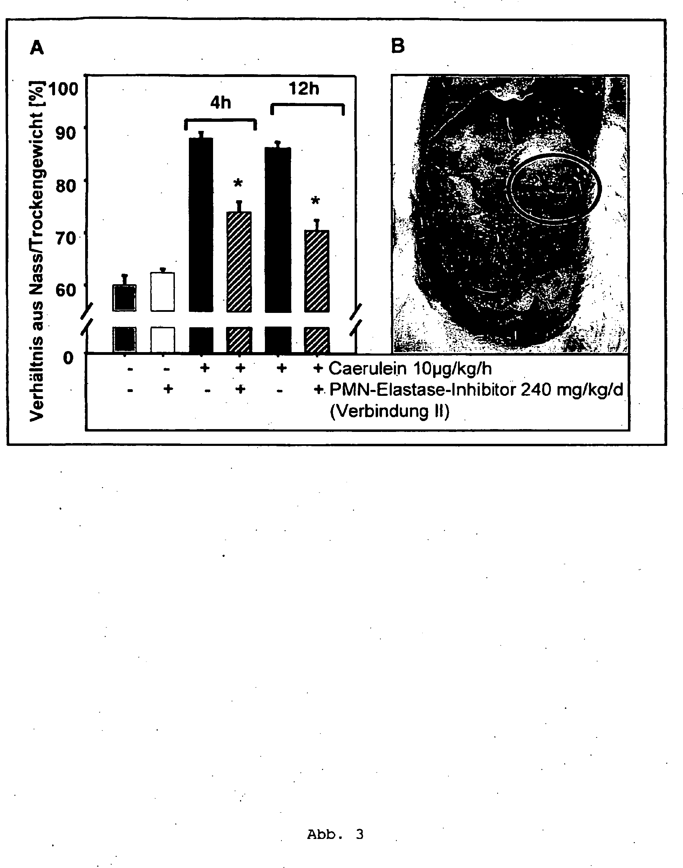

**Abbildung 3** zeigt die Beurteilung des Pankreasödem: **Abbildung 3A** zeigt den Wassergehalt des Pankreas. **Abbildung 3B** zeigt die makroskopischen Befunde des Pankreas nach supramaximaler Caeruleinstimulation. Auffällig ist die gelatinöse Masse des Pankreas (mit einem Kreis markiert), die einen Wassergehalt von 90% aufweist.

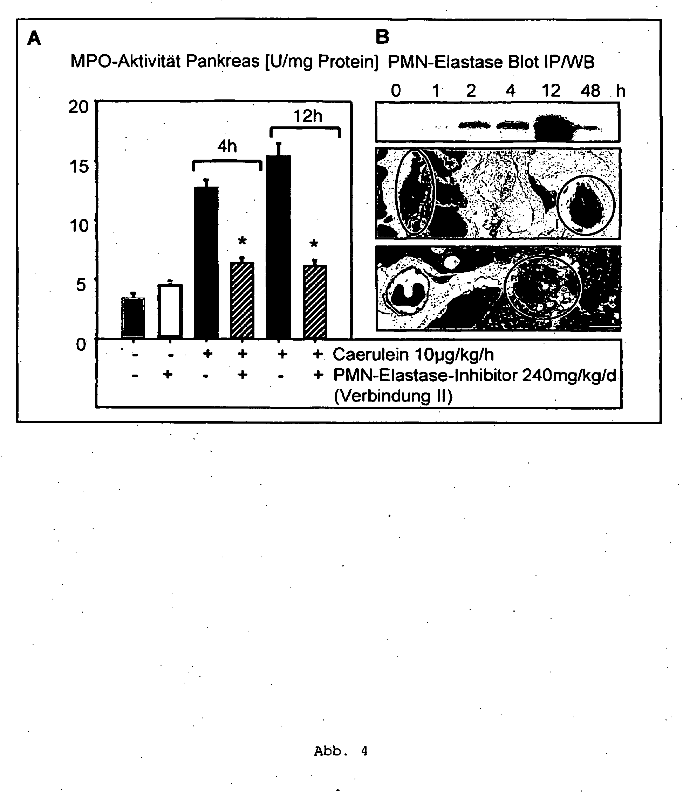

**Abbildung 4** zeigt die Messung der Myeloperoxidaseaktivität (MPO) in pankreatischem Gewebe nach supramaximaler Caeruleinstimulation. Nach vier und zwölf Stunden zeigt sich ein hochsignifikanter Anstieg der Enzymaktivität **(****Abbildung 4A****).** Die maximale Aktivität wurde nach zwölf Stunden festgestellt. Die Balken zeigen die Durchschnittswerte in mU MPO Aktivität / mg pankreatisches Protein ± SEM (SEM: Standardabweichung der Probenverteilung: σₓ= √1/N ∑ (xᵢ-x)2), von fünf Tieren pro Zeitintervall an. Die Western-Blot-Analyse zeigt einen stetigen Anstieg der Leukozytenelastase-Expression im Pankreas **(****Abbildung 4B****)**. Die ultrastrukturelle Analyse des Pankreasgewebes von Tieren, die mit Caerulein für eine Stunde inkubiert wurden, zeigt eine Transmigration von Neutrophilen aus Gefäßen in den interstitiellen Raum zwischen den Pankreasazini-Zellen.

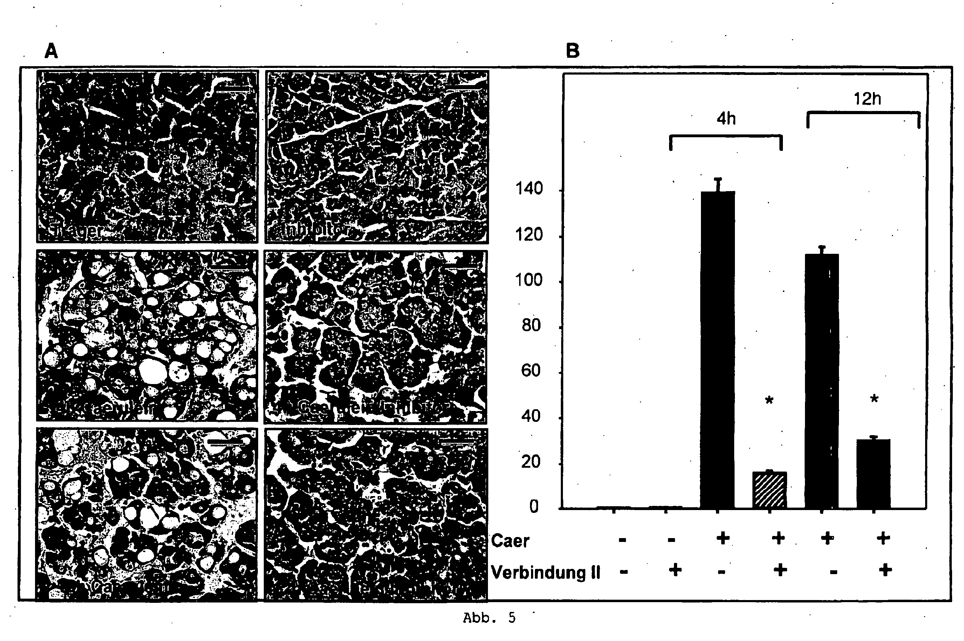

**Abbildung 5** zeigt lichtmikroskopische Aufnahmen des Pankreasgewebes (5A).
   Die Quantifizierung der Anzahl von Vakuolen zeigt eine hochsignifikante Verminderung auf ungefähr 15 bis 25%.

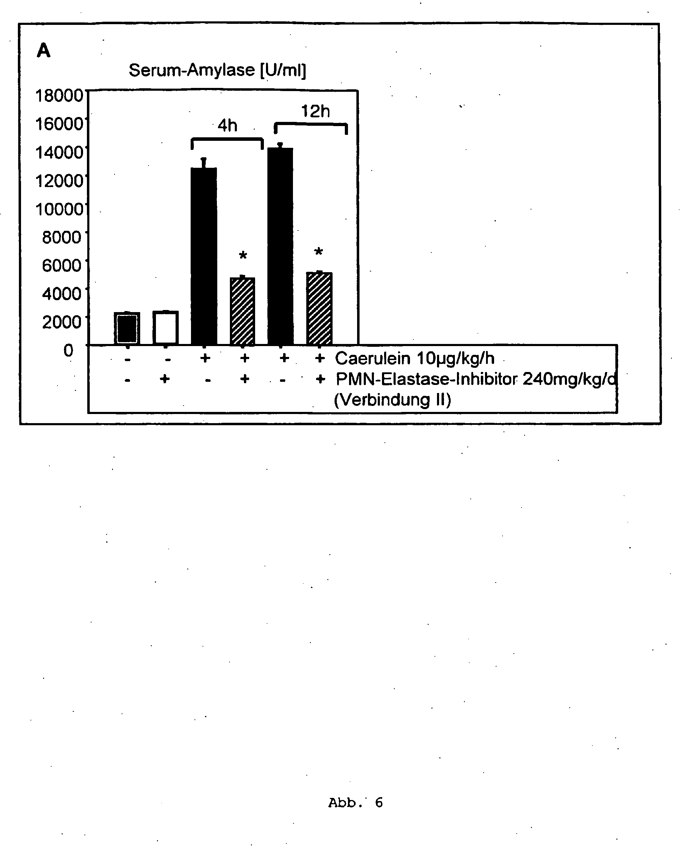

**Abbildung 6** zeigt die Serumamylaseaktivität.

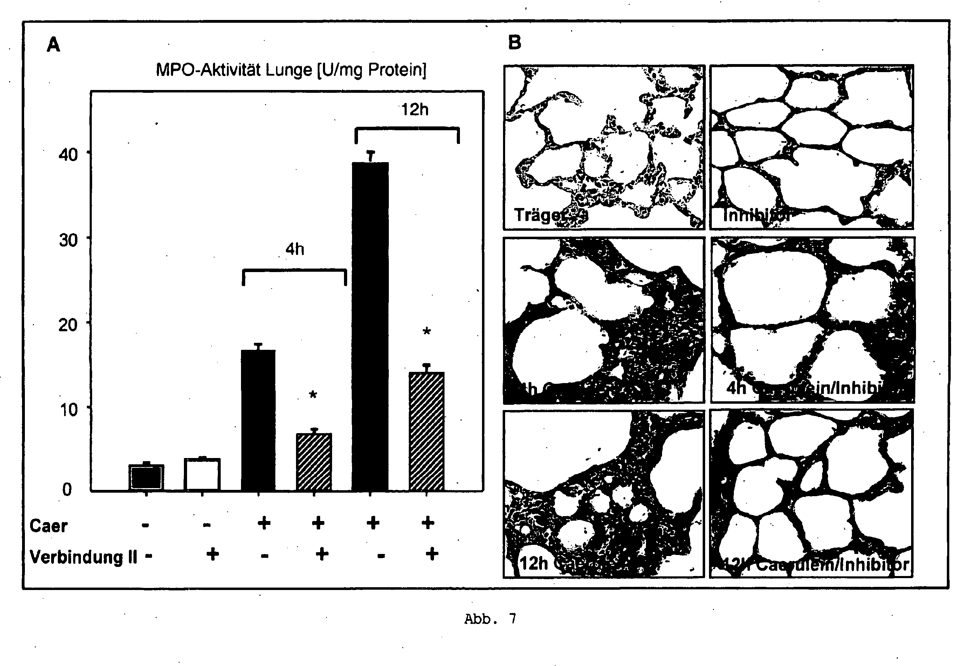

**Abbildung 7** zeigt lichtmikroskopische Aufnahmen des Lungengewebes (7B). Die Messung der Myeloperoxidase-Aktivität (MPO) in pankreatischem Gewebe nach supramaximaler Caeruleinstimulation in den angegebenen Zeitintervallen zeigt nach vier und zwölf Stunden einen hochsignifikanten Anstieg der enzymatischen Aktivität (7A). Die Balken zeigen Durchschnittswerte in mU MPO-Aktivität pro mg Lungenprotein ± SEM von fünf Tieren zu jedem Zeitpunkt.

### Beispiele:

### Verwendete Materialien:

Caerulein wurde von Pharmacia, Freiburg, Deutschland, erhalten. Humane neutrophile Elastase wurde von Calbiochem (San Diego, CA, U.S.A.) bezogen. Dieses kommerzielle Produkt wurde aus humanem Serum durch HPLC aufgereinigt (Proteinkonzentration > 20 Einheiten/mg Protein spezifische Aktivität; in 50 mM Natriumacetat pH 5,5 und 200 mM Natriumchlorid; Reinheit > 95%). Humane Myeloperoxidase wurde von Calbiochem bezogen und aus Blut mit HPLC aufgereinigt (1 mg/10 ml Proteinkonzentration, 150 bis 200 Einheiten mg Protein spezifische Proteinaktivität, in 100 mM Natriumchlorid, 50 mM Natriumacetat, Reinheit > 95%). Schweinepankreas-Elastase wurde von Calbiochem bezogen und wurde aus Schweinepankreas aufgereinigt. Die spezifische Aktivität war 50 Einheiten/mg Protein in 50 mM Kaliumphosphatpuffer, pH 7,0. Cathepsin B wurde von Calbiochem bezogen. Dieses kommerzielle Produkt wurde aus humaner Leber mit HPLC aufgereinigt (1,136 mg/ml Proteinkonzentration, 22 Einheiten/mg Protein spezifische Aktivität; in 20 mM Natriumacetat (pH 5,0) und 1 mM EDTA; Reinheit > 95%). Vor der Verwendung wurde Cathepsin B mit 1 mM Ditiotreithol (DTT) für 30 Minuten auf Eis aktiviert. Cathepsin L wurde von Calbiochem bezogen und aus humaner Leber mit einer Konzentration von 1584 mU/mg Protein präpariert. Die Substrate, die für die Analysen verwendet wurden, waren [CBZ-Ile-Pro-Arg]₂-Rhodamin 110 und [CBZ-Arg₂]-Aminomethyl-Coumarin (AMC) von Molecular Probes (Eugene, Oregon, U.S.A.), [CBZ-Ala₄]- Aminomethyl-Coumarin (AMC) von Molecular Probes sowie [CBZ-Phe-Arg]-Rhodamin 110 (R110) (Eugene Orgeon U.S.A.). Alle weiteren Chemikalien wurden in der höchsten Reinheit entweder von Sigmar-Aldrich (Eppelheim, Deutschland) oder Merck (Darmstadt, Deutschland), Amersham Pharmacia Biotech (Buckinghamshire, UK) oder Bio-Rad (Hercules, Kalifornien, U.S.A.) bezogen.

Die Tiere wurden in den Charles-River-Breeding-Laboratorien (Sulzbach, Deutschland) gezüchtet. Alle Tierexperimente wurden in Übereinstimmung mit den Richtlinien der lokalen Tierverwendungs- und Tierschutzvorschriften ausgeführt.

### Beispiel 1

### In vitro Analyse der Inhibitor Spezifität und Kapazität

Die Aktivität der neutrophilen Elastase, pankreatischen Elastase, von Trypsin, Cathepsin L, Cathepsin B, und Myeloperoxidase wurde in Abwesenheit und Anwesenheit von verschiedenen Konzentrationen der Verbindung II bestimmt (die Konzentrationen lagen im Bereich von 1 nM bis 1 mM). Die Trypsinaktivität (10 mU), Myeloperoxidaseaktivität (100 mU) und die neutrophile Elastase-Aktivität (10 mU) wurde durch die Verwendung der spezifischen fluorogenen Substrate [CBZ-Ile-Pro-Arg]₂-Rhodamin 110 oder [CBZ-Ala₄]-Aminomethyl-Coumarin (AMC) in 100 mM Tris-HCl-Puffer (pH 8,0), der 5 mM CaCl₂, 10 µM Substrat (Endkonzentration) und 10 mU bovines Trypsin in einem Volumen von 150 µl enthielt, bei einer Exzitationswellenlänge von 485 nm und einer Emissionswellenlänge von 530 nm oder einer Exzitationswellenlänge von 350 nm und einer Emissionswellenlänge von 460 nm bei 37 °C bestimmt. Die Anfangsraten der Substrathydrolyse wurden als arbiträre Fluoreszenzeinheiten pro Minute gemessen. Nachfolgend wurde die Cathepsin B Aktivität (100 mU) und Cathepsin L Aktivität (100 mU) in 0,25 M Natriumacetat-Puffer (pH 5,0), der 2 mM EDTA 1 mM DTT, 10 µM des spezifischen Substrates CBZ-[Arg]₂-Aminomethyl-Coumarin oder 10 µM [CBZ-Phe-Arg]-Rodamin 110 (Endkonzentration) in einem Endvolumen von 150µl enthielt, bei einer Exzitationswellenlänge von 350 nm und einer Emissionswellenlänge von 460 nm oder 485 nm und einer Emissionswellenlänge von 530 nm bei 37 °C bestimmt.

Die Spaltung der Substrate wurde für 60 Minuten in einem Mikroplattenfloureszenzlesegerät (SPECTRAmax GEMINI, Molecular Devices, Sunnywell, Kalifornien, U.S.A.) gemessen und zur Ausgangsaktivität in Abwesenheit der Verbindung II verglichen. Die Ergebnisse wurden in Prozent der Ausgangsenzymaktivität berechnet (Kukor et al., 2002).

Die Ergebnisse sind in Abbildung 1 dargestellt. Die Verbindung II inhibiert Cathepsin B und L, die Myeloperoxidase und Trypsin nicht. Die inhibitorische Wirksamkeit der Verbindung II zur Inhibierung der humanen neutrophilen Elastase war fünffach höher im Vergleich zur pankreatischen Elastase.

### Beispiel 2

### Induktion einer akuten Caerulein-induzierten Pankreatitis

Männliche Wistar-Ratten (140 bis 250 g) wurden mit Pentobarbital (30 mg/kg) anästhesiert. Eine Kanüle wurde in der Jugularvene plaziert und den Tieren wurde per Infusion supramaximale Konzentration von Caerulein (10 µg/kg pro Stunde) für vier und zwölf Stunden verabreicht. Männliche Wistar-Ratten (250 bis 300 g) erhielten eine Sondenernährung der Verbindung II (mit einer Konzentration von 240 mg/kg pro Tag) drei und eine Stunde vor der Induktion der CaeruleinPankreatitis für vier und zwölf Stunden. Tiere, die eine Kochsalzlösung als Infusion erhielten und mit der Verbindung II über eine Sonde gefüttert wurden, dienten als Kontrolle. Jede Behandlungsgruppe bestand aus fünf Tieren. Nach dem Ausbluten unter Etheranästhesie wurde der Pankreas schnell entfernt, von Fett befreit und Gewebeblöcke wurden in 5% Paraformaldehyd für die Histologie in Paraffin eingebetteten Schnitten fixiert. Der größte Anteil des Pankreas wurde in flüssigem Stickstoff gefroren und bei -80 °C für die spätere Proteinanalyse und zur Detektion der Enzymaktivität aufbewahrt.

Um die Fähigkeit der Verbindung II zur Verminderung der lokalen und systemischen Schäden als Folge einer Pankreatitis zu untersuchen, wurden Gruppen von jeweils fünf männlichen Wistar-Ratten wie folgt verwendet: Kontrolle 1 (Trägerlösung), Kontrolle 2 (Verbindung II), supramaximale Konzentration von Caerulein (10 µg/kg pro Stunde) für vier und zwölf Stunden, supramaximale Menge von Caerulein + orale Fütterung der Verbindung II mit einer Konzentration von 240 mg/kg pro Tag, wobei die Verbindung II durch Sondenfütterung oral jeweils drei Stunden und eine Stunde vor der Induktion der CaeruleinPankreatitis verabreicht wurde und in 100 µl Polyethylenglykol 400 gelöst war. Die Tiere wurden nach vier und zwölf Stunden getötet und pankreatisches Gewebe, Serum und Lungengewebe wurde für die weiteren Analysen gesammelt (s. Abbildung 2).

### Beispiel 3

### Auswirkung der Verbindung II auf das Ausmaß von pankreatischen Ödemen

Bestimmung des pankreatischen Wassergehalts: Der Wassergehalt des Pankreas wurde durch Vergleich des frisch gewonnenen Gewebes (Naßgewicht) mit dem Gewicht derselben Probe nach Austrocknung (Trockengewicht) bestimmt. Um eine vollständige Austrocknung zu gewährleisten, wurde das Gewebe bei 160 °C für 24 Stunden inkubiert, wobei ein konstantes Gewicht erreicht wurde. Die Ergebnisse wurden als Prozentsatz des Unterschiedes zwischen dem Naßgewicht und dem Trockengewicht geteilt durch das ursprüngliche Naßgewicht dargestellt.

Eine Verabreichung der Verbindung II allein führte nicht zu einer signifikanten Änderung im Wassergehalt des Pankreas. Die als Kontrolle 1 behandelten Tiere zeigten einen Wassergehalt von ungefähr 60% der sich nicht signifikant von der Gruppe unterschied, die oral mit der Verbindung II gefüttert wurde (Kontrolle 2).

Die Caerulein-Infusion induzierte ein pankreatisches Ödem, was sich in einer Steigerung des Wassergehalts auf bis zu 90% wiederspiegelte. Die orale Applikation der Verbindung II zeigte eine signifikante Verminderung in der Bildung von Ödemen sowohl nach vier als auch nach zwölf Stunden des Beginns der Caerulein-Infusion (Abbildung 3). Das rechte Bild in der Abbildung 3 zeigt den makroskopischen Befund im Rattenabdomen nach der Induktion der ödematösen Pankreatitis.

### Beispiel 4

### Bestimmung der Myeloperoxidase-Aktivität in pankreatischen Homogenaten

Zur Messung der Myeloperoxidase (MPO) Aktivität wurde das Gewebe aufgetaut und auf Eis in 20 mM Kaliumphosphatpuffer (pH 7,4) homogenisiert und für 10 Minuten bei 20.000 g bei 4°C zentrifugiert. Das Pellet wurde in 50 nM Kaliumphosphatpuffer (pH 6,0), der 0,5% Cethyltrimethylamoniumbromid enthielt, resuspendiert. Die Suspension wurde viermal eingefroren und wieder aufgetaut, einmal für 10 Sekunden bei einer Leistungsstufe von 30% mit Schallwellen behandelt und für 10 Minuten bei 4°C bei 20.000 g zentrifugiert. Die MPO-Aktivität wurde nach Mischung von 50 µl Überstand in 200 µl eines 50 mM Käliumphosphatpuffers (pH 6,0), der 0,53 mM O-Dianisidin und 0,15 mU H₂O₂ enthielt, untersucht.

Der ursprüngliche Anstieg der Absorption bei 450 nm wurde bei Raumtemperatur mit einem Dynatech MR 5000 ELISA-Lesegerät gemessen. Die Ergebnisse wurden als Einheiten von MPO dargestellt, wobei eine Einheit 1 µM H₂O₂ pro Minute pro mg pankreatisches Protein oxidiert. Die Balken zeigen Durchschnittswerte in mU MPO-Aktivität/mg pankreatisches Protein von fünf Tieren pro Zeitpunkt (s. Abb. 4).

Mit der Messung der Myeloperoxidase-Aktivität sollte die Anzahl von Neutrophilen bestimmt werden, die in das pankreatische Gewebe eindringen. Eine direkte Interaktion von der Verbindung II mit MPO wurde in *in vitro* Versuchen ausgeschlossen, um sicherzustellen, daß die verringerten Werte von MPO zu einer verminderten neutrophilen Infiltration korrelierten und nicht durch die Inhibierung der MPO verursacht wurden. Die Tiere, die mit der Verbindung II behandelt wurden, zeigten eine mehr als 50%ige Verringerung der MPO-Aktivität in pankreatischem Gewebe.

Da die MPO-Aktivität im pankreatischen Gewebe nur einen indirekten Nachweis für das Vorhandensein von Neutrophilen darstellt, wurden ebenfalls Immunopräzipitations-Experimente für den Zeitverlauf der Caeruleinpankreatitis bis zu 48 Stunden durchgeführt, und die Proben wurden auf Expression der neutrophilen Elastase mittels Westernblot analysiert.

### Immunopräzipitation und Immunoblotting:

Das pankreatische Gewebe wurde in eisgekühltem PBS oder Triton X 100 Lysispuffer, der Proteaseinhibitoren (1 ml/mg Gewebe), 10 µg/ml Aprotinin, 10 µg/ml Leupeptin, 0,01 M Natriumpyrophosphat, 0,1 M Natriumflourid, 1 mM Dihydrogenperoxid, 1 mM L-Phenyl-Methyl-Sulfunyl-Flourid PMSF und 0,02 % Sojabohnentrypsinihibitor) enthielt, mit einem Dounce S Glas Homogenisierer (Braun-Melsungen), für Westernblots oder ohne Inhibitoren für die Detektion der enzymatischen Aktivitäten homogenisiert. Die Proteinkonzentration wurde durch einen modifizierten Bradford-Assay (Bio-Rad) bestimmt und jeweils gleiche Mengen von Protein wurden in aufeinanderfolgenden Experimenten eingesetzt. Für die Immunopräzipitiation wurde eine Mischung von Protein A und G-Sepharose (Amersham Pharmacia Biotech) mit Antikörper in 20 mM HEPES pH 7,4 vorinkubiert.

Die Lysate wurden mit Ratten Non-Immunserum vorgereinigt, zu den gekoppelten Antikörpern zugegeben und für eine Stunde bei 4 °C auf einer Drehscheibe inkubiert. Die Präzipitate wurden mit HNTG (HNTG: HEPES-NaCl-Triton-Glycerin Puffer) gewaschen und für fünf Minuten in zweifach SDS-Probenpuffer gekocht. SDS-Polyacrylamidgelelektrophorese wurde in einem diskontinuierlichen Puffersystem durchgeführt, und die Gele wurden auf Nitrozellulosemembranen (Highbond C, Amersham Pharmacia Biotech) geblottet. Nach einer Blockierung in NET-Gelatine 0,2% (NET: Natrium-EDTA-Tris Puffer) über Nacht wurde die Immunoblotanalyse durchgeführt, gefolgt von einer Chemolumineszenzdetektion (Amersham Pharmacia Biotech), wobei Meerettichperoxidase gekoppelt an einen Schaf-Anti-Maus IgG (Amersham Pharmacia) oder Ziegen-Anti-Kaninchen IgG (Amersham Pharmacia Biotech) verwendet wurde.

Die neutrophile Elastaseexpression zeigte ihr Maximum nach zwölf Stunden, hatte aber bereits nach einer Stunde nach Beginn der Pankreatitis eine signifikante Steigerung. Ebenfalls nach einer Stunde nach Beginn der CaeruleinPankreatitis konnte die Transmigration von Neutrophilen aus kleinen Gefäßen in den interstitiellen Raum des Pankreas nachgewiesen werden, wie durch die Elektronenmikroskopie dargestellt wurde (s. Abbildung 4).

Für die Elektronenmikroskopie wurden kleine Blöcke (2 mm im Durchmesser) des pankreatischen Gewebes des Rattenpankreas nach der supramaximalen Caerulein-Infusion und von Kontrolltieren in 2% Formaldehyd/2% Glutarldehyd fixiert, in Epon eingebettet und Osmium, Uranyl und Blei kontrastierte dünne Schnitte wurden für die Elektronenmikroskopie eingesetzt. Ultradünne Schnitte (60 nm) wurden mit Hilfe eines Leica Ultramikrotoms hergestellt. Die Proben wurden in einem Philips 400 Elektronenmikroskop untersucht und bei einer 30.000-fachen Vergrößerung fotografiert.

### Beispiel 5

### Auswirkung der Verbindung II auf morphologische Schäden

Die supramaximale Stimulation mit Caerulein führte zu einem interstitiellen Ödem und der Bildung von großen intrazellulären Vakuolen in pankreatischen Azinazellen. Die Verabreichung der Verbindung II führte nicht zu irgendwelchen morphologischen Veränderungen, wie sie in einer akuten Pankreatitis beobachtet werden. Die Verabreichung der Verbindung II zusammen mit Caerulein verhinderte die Entwicklung des interstitiellen Ödems und der intrazellulären Vakuolisation. Die noch nachweisbaren Vakuolen waren sehr viel kleiner, und die Anzahl der infiltrierenden entzündlichen Zellen wurde stark vermindert. Die Quantifizierung der Anzahl von Vakuolen in den verschiedenen Behandlungsgruppen zeigte eine hochsignifikante Verminderung auf ungefähr 15 bis 25% der Vakuolen, die bei der unbehandelten Pankreatitis gefunden wurden (s. Abbildung 5).

### Beispiel 6

### Auswirkung der Verbindung II auf die Hyperamylasämie

Als ein Ergebnis einer Zellschädigung kann eine gesteigerte Amylase-Aktivität im Serum während des Verlaufs der Caerulein-induzierten Pankreatitis bereits nach einer Stunde nach Beginn der Pankreatitis nachgewiesen werden. Das Ausmaß der pankreatischen Azinuszell-Zerstörung korreliert zum Ausmaß der Amylase-Aktivität im Serum. Es wurde gezeigt, daß durch die orale Verabreichung der Verbindung II die Amylase-Aktivität, die im Serum der Tiere nach vier und zwölf Stunden der Pankreatitis nachgewiesen werden konnte, signifikant vermindert wurde (s. Abbildung 6).

### Beispiel 7

### Auswirkung der Verbindung II auf die Myeloperoxidase-Aktivität in der Lunge während einer Pankreatitis

Die Pankreatitis beeinflußt nicht nur das Pankreas sondern führt auch zu einer systemischen Entzündungsantwort, die andere Organe, wie die Nieren oder die Lunge betrifft. Um zu untersuchen, ob die Verbindung II eine Rolle bei der Beeinflussung der extrapankreatischen Effekte des Krankheitsverlaufs spielt, wurde die Myeloperoxidase-Aktivität in Lungengewebe untersucht, um das Ausmaß der Leukozyteninfiltration zu beurteilen, sowie die morphologischen Unterschiede in den Lungen von Pankreatitis-Tieren und unbehandelten Kontrollen zu analysieren. Die morphologischen Unterschiede in den Lungengeweben während einer Caerulein-induzierten Pankreatitis bestanden aus Alveolarflüssigkeitsansammlungen und einer progressiven Verdickung, Hyperämie und neutrophiler Infiltration des intraalveolaren Gewebes. All diese Ereignisse wurden durch die Verabreichung der Verbindung II reduziert. Die Tiergruppe, die mit der Verbindung II behandelt wurde, zeigte eine signifikante Verminderung der MPO-Aktivität, welche zu einer reduzierten Anzahl von Neutrophilen, die das Lungengewebe infiltrieren, korrelierte. Der beobachtete Effekt war zwölf Stunden nach Ausbruch der Pankreatitis größer als nach vier Stunden. Diese Beobachtung stimmt mit der Tatsache überein, daß die systemische Entzündungsreaktion nach zwölf Stunden des Verlaufs einer akuten Pankreatitis ihren Höhepunkt erreicht. Diese Daten zeigen deutlich, daß die Inhibierung der neutrophilen Elastase durch einen oral-verabreichbaren Wirkstoff nicht nur die lokalen pankreatischen Schäden reduziert, sondern ebenfalls die systemische Entzündungsreaktion während der Pankreatitis beeinflußt (s. Abbildung 7).

### Literatur:

Ammann RW, Akrovbiantz A, Largiader F, Schueler G. Course and outcome of chronic pancreatitis. Longitudinal study of a mixed medicalsurgical series of 245 patients. Gastroenterology 1984; 86:820-828.
Cavallini G, Tittobello A, Frulloni L, Masci E, Mariani A, Di Francesco V. Gabexate for the prevention of pancreatic damage related to endoscopic retrograde cholangiopancreatography. The New England J. of Med. 1996; 335(13): 919-923.
Dominguez-Munoz JE, Carballo F, Garcia MJ, de Diego JM, Rabago L, Simon. MA, de la Morena J. Clinical usefulness of polymorphonuclear elastase in predicting the severity of acute pancreatitis: results of a multicentre study. Br. J. Surg 1991; 78:1230-1234.
Geokas MC, Murphy R, McKenna RD. The role of elastase in acute pancreatitis. I. Intrapancreatic elastolytic activity in bile-induced acute pancreatitis in dogs. Arch. Pathol. 1968; 86:117-126.
Kukor Z, Mayerle J, Krüger B, Toth M, Steed P, Halangk W, Lerch MM, Sahin-Toth M. Presence of cathepsin B in the human pancreatic secretory pathway and ist role in trypsinogen activation during hereditary pancreatitis. J.Biol.Chem. 2002; 277:21389-21396.
Withcomb DC, Gorry MC, Preston RA, Furey W, Sossenheimer MJ, Ulrich CD, Martin SP, Gates LK, Amann ST, Toskes PP, Liddle R, McGrath K, Uomo G, Post JC, Ehrlich GD. Hereditary pancreatitis is caused by a mutation in the cationic trypsinogen gene. Nat. Genet. 1996; 14:141-145.

## Patentansprüche

1. Verwendung eines Peptidyl-Trifluormethylketons oder seines Solvats zur Herstellung einer pharmazeutischen Zusammensetzung zur Vorbeugung und Behandlung einer Pankreatitis, wobei das Peptidyl-Trifluormethylketon folgende Struktur hat:

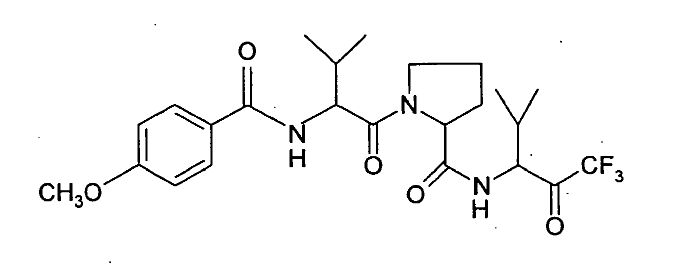

2. Verwendung nach Anspruch 1, **dadurch gekennzeichnet, daß** das Peptidyl-Trifluormethylketon folgendes Stereoisomer ist:

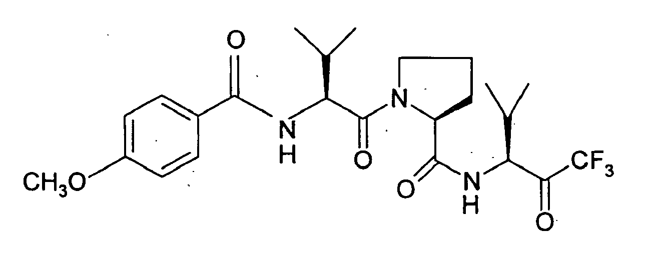

3. Verwendung nach einem der Ansprüche 1 oder 2, **dadurch gekennzeichnet, daß** die Pankreatitis eine chronische oder chronische rezidivierende Pankreatitis ist.

4. Verwendung nach einem der Ansprüche 1 oder 2, **dadurch gekennzeichnet, daß** die Pankreatitis eine post-ERCP Pankreatitis ist.

5. Verwendung nach einem der Ansprüche 1 bis 4, **dadurch gekennzeichnet, daß** das Peptidyl-Trifluormethylketon oral verabreicht wird.

6. Verwendung nach einem der Ansprüchen 1 bis 5, **dadurch gekennzeichnet, daß** die Verwendung den Einsatz eines pharmazeutisch geeigneten Trägermaterials umfaßt.

7. Verwendung nach einem der Ansprüchen 1 bis 6, **dadurch gekennzeichnet, daß** sie zur Vorbeugung einer Pankreatitis erfolgt.

8. Verwendung nach einem der Ansprüchen 1 bis 7, **dadurch gekennzeichnet, daß** sie zur Vorbeugung einer post-ERCP Pankreatitis erfolgt.

9. Peptidyl-Trifluormethylketon oder Solvat davon zur Verwendung bei Vorbeugung und Behandlung einer Pankreatitis, wobei das Peptidyl-Trifluormethylketon folgende Struktur hat:

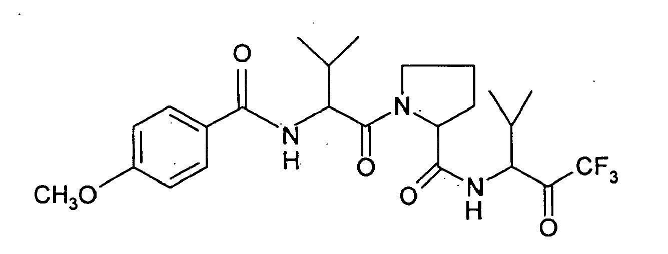

## Claims

1. Use of a peptidyl trifluoromethyl ketone oder a solvate thereof for the preparation of a pharmaceutical composition for prevention and treatment of pancreatitis, wherein the peptidyl trifluoromethyl ketone has the following structure:

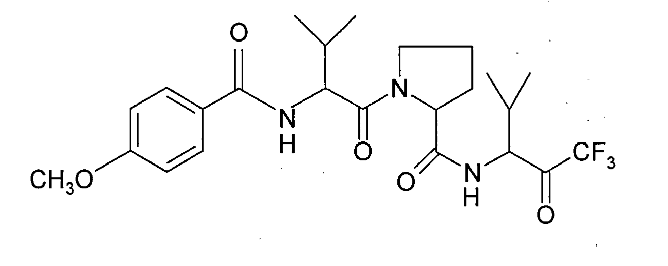

2. Use according to claim 1, wherein the peptidyl trifluoromethyl ketone is the following stereoisomer:

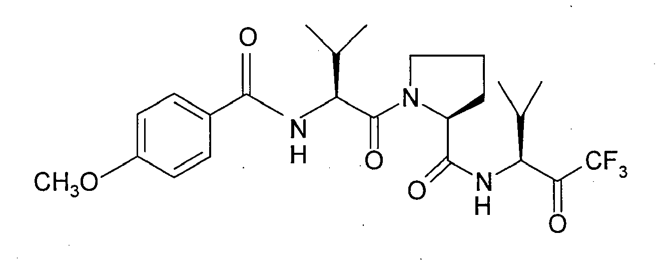

3. Use according to any of claims 1 or 2, wherein the pancreatitis is a chronic or chronically recurrent pancreatitis.

4. Use according to any of claims 1 or 2, wherein the pancreatitis is a post-ERCP pancreatitis.

5. Use according to any of claims 1 to 4, wherein peptidyl trifluoromethyl ketone is orally administered.

6. Use according to any of claims 1 to 5, wherein the use comprises use of a pharmaceutically acceptable carrier material.

7. Use according to any of claims 1 to 6, wherein the use is use for prevention of pancreatitis.

8. Use according to any of claims 1 to 7, wherein the use is use for prevention of post-ERCP pancreatitis.

9. Peptidyl trifluoromethyl ketone oder a solvate thereof for use in prevention and treatment of pancreatitis, wherein the peptidyl trifluoromethyl ketone has the following structure:

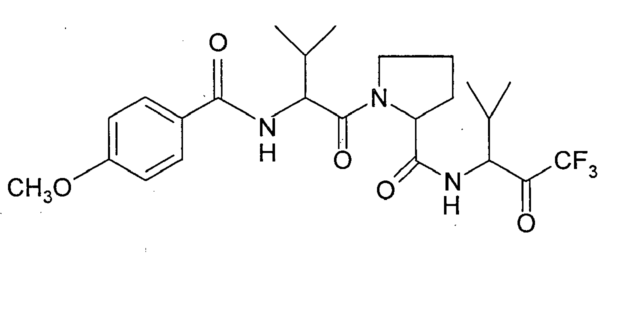

## Revendications

1. Utilisation d'une peptidyl-trifluorométhyl-cétone, ou d'un solvat de cette cétone, en vue de la fabrication d'une composition pharmaceutique conçue pour la prévention et le traitement d'une pancréatite, laquelle peptidyl-trifluorométhyl-cétone présente la structure suivante :

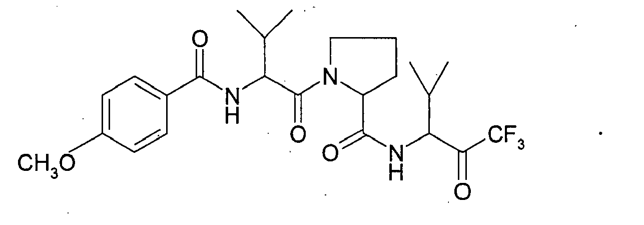

2. Utilisation conforme à la revendication 1, **caractérisée en ce que** la peptidyl-trifluorométhyl-cétone est le stéréoisomère suivant :

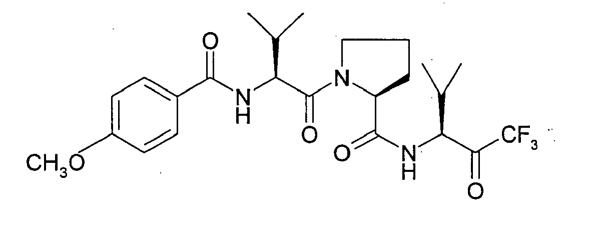

3. Utilisation conforme à l'une des revendications 1 et 2, **caractérisée en ce que** la pancréatite est une pancréatite chronique ou une pancréatite chronique récidivante.

4. Utilisation conforme à l'une des revendications 1 et 2, **caractérisée en ce que** la pancréatite est une pancréatite post-CPRE.

5. Utilisation conforme à l'une des revendications 1 à 4, **caractérisée en ce que** la peptidyl-trifluorométhyl-cétone sera administrée par voie orale.

6. Utilisation conforme à l'une des revendications 1 à 5, **caractérisée en ce qu'**elle comporte le recours à un véhicule pharmacologiquement admissible.

7. Utilisation conforme à l'une des revendications 1 à 6, **caractérisée en ce qu'**elle sert à la prévention d'une pancréatite.

8. Utilisation conforme à l'une des revendications 1 à 7, **caractérisée en ce qu'**elle sert à la prévention d'une pancréatite post-CPRE.

9. Peptidyl-trifluorométhyl-cétone, ou un solvat de cette cétone, pour une utilisation dans la prévention et le traitement d'une pancréatite, laquelle peptidyl-trifluorométhyl-cétone présente la structure suivante :
